# EUROPEAN PATENT SPECIFICATION

(11) **EP 0 335 336 B1**
(45) Date of publication and mention of the grant of the patent: **04.08.1993**
(21) Application number: 89105468.6
(22) Date of filing: 28.03.1989
(51) Int. Cl.: C07C 209/72, C07C 211/36

(54) **Catalytic hydrogenation of crude methylene bridged polyphenylamines to produce polycyclohexylamines**
Katalytische Hydrierung von rohen methylenüberbrückten Polyphenylenaminen zur Herstellung von Polycyclohexylaminen
Hydrogénation catalytique de polyphénylamines brutes à ponts méthylène pour produire des polycyclohexylamines

(30) Priority: 30.03.1988 US 175010
(43) Date of publication of application: 04.10.1989
(73) Proprietor: AIR PRODUCTS AND CHEMICALS, INC., Trexlertown Pennsylvania 18087 (US)
(72) Inventor: Vedage, Gamini Ananda, Bethlehem, PA 18017 (US); Henderson, William Weldon, Allentown, PA 18104 (US); Toseland, Bernard Allen, Allentown, PA 18104 (US)
(74) Representative: Schwabe - Sandmair - Marx

(56) References cited:
- EP-A- 0 001 425
- EP-A- 0 231 788
- FR-A- 2 372 142
- GB-A- 1 351 487
- US-A- 3 959 374

## Description

This invention pertains to an improved process for hydrogenating methylene bridged polyphenylamines to the polycyclohexylamines counterparts.

There is substantial literature in the art with respect to the hydrogenation of methylene bridged polyphenylamines to produce polycyclohexylamine counterparts using a hydrogenation catalyst. A material for which there has been substantial hydrogenation activity effort is methylenedianiline with the end product being bis(4-aminocyclohexyl)methane; it is also called bis(para-aminocyclohexyl)methane or PACM. Some of the early work was done by Whitman and Barkdoll, et al. and their work is set forth in a series of U.S. Patents, e.g., 2,511,028; 2,606,924; 2,606,925; and 2,606,928. Basically the processes described in these patents involve the hydrogenation of methylenedianiline at pressures in excess of 1,38MPag (200 psig), preferably in excess of 6,89MPag (1,000 psig), at temperatures within a range of 80 to 275°C utilizing a ruthenium catalyst for the hydrogenation. The hydrogenation is carried out under liquid phase conditions. Usually an inert organic solvent is used in the hydrogenation process. Examples of ruthenium catalysts utilized for the hydrogenation process include ruthenium oxides such as ruthenium sesquioxide and ruthenium dioxide; and ruthenium salts.

Brake, et al. continued with the development of hydrogenation process to produce PACM and they found that if the ruthenium catalyst was carried upon a support and the support alkali-moderated, the catalyst was much more active and catalytically effective in producing the desired hydrogenated PACM product. Alkali moderation was effected by contacting the catalyst and support with alkali metal hydroxide or an alkoxide; also, such alkali moderation of the catalyst could be effected prior to hydrogenation or in situ during the hydrogenation. Representative patents showing the utilization of alkali-moderated ruthenium catalysts to hydrogenate methylenedianiline include U.S. 3,636,108; 3,644,522; and U.S. 3,697,449. Alkali metal and alkaline earth metal nitrates and sulfates have similarly been shown effective in U.S. 4,448,995 under high pressure 27,6MPag (4000 psi)) hydrogenation conditions.

Other catalysts have been utilized for the hydrogenation of methylenedianiline and examples are shown in U.S. 3,591,635 and U.S. 3,856,862. Both patents disclose the use of rhodium as a catalytic material and each require the use of an aliphatic alcohol as a solvent. The rhodium is alkali moderated using ammonium hydroxide as a pretreatment or by carrying out the reaction in the presence of ammonia. Also, in European application 66,212 rhodium on alumina in the presence of butyl ether is disclosed to obtain 15-40% trans,trans- isomer ratio contents, but again the pressures are high 27,6MPag (4000 psi)) and the reaction times short, leading to difficult reaction product control.

In the catalytic processes for hydrogenating methylene-bridged polyphenylamines as described above, the bridged polyphenylamines were purified, i.e., distilled to remove trace impurities and oligomers. Crude polyphenylamines were difficult to hydrogenate presumably because impurities in the feedstock poisoned the catalyst. U.S. 3,959,374 describes a process for the catalytic hydrogenation of methylene bridged polyphenylamine which contained trace impurities and oligomers. More specifically a crude methylenedianiline feed containing these impurities and oligomers is initially treated with hydrogen in the presence of a nickel-containing hydrogenation catalyst prior to hydrogenation in the presence of a ruthenium catalyst. The pretreatment overcomes low yields (52.4%) and long reaction times associated with nickel and cobalt. In the absence of the pretreatment, ruthenium catalysts, although commonly used for hydrogenation of purified methylenedianiline, were not suited for hydrogenation of a methylene dianiline feed containing impurities, e.g., isomeric impurities.

EP-A 0 231 788 discloses a process for the catalytic hydrogenation of bis-(4-aminophenyl-)methane to the corresponding bis-(4-aminocyclohexyl-)methane wherein the product contains from about 15 to 40 % by weight of the trans,trans-isomer. In said process, the catalytic hydrogenation is effected in the presence of a catalytic system comprising two transition metals, i. e. rhodium und ruthenium.

EP-A 0 001 425 discloses a process wherein a ruthenium carrier catalyst is used. A material on the basis of hydroxides and/or oxide hydrates of chromium and manganese and/or their dihydration products is used as the catalyst carrier wherein the catalyst contains anorganic and/or organic alkali metal compounds. Such a process is employed for the catalytic hydrogenation of aromatic amines, in particular of condensation products of aniline with formaldehyde. However, the hydrogenation is carried out in one step and without that a rhodium hydrogenation catalyst is included.

This invention relates to an improved process for the catalytic hydrogenation of methylene -bridged polyphenylamines to produce the polycyclohexylpolyamine counterparts using a catalyst containing rhodium metal for the hydrogenation. The improvement in the hydrogenation process to produce a polycyclohexylamine counterpart is achieved by initially contacting in a first hydrogenation zone, the crude methylene bridged polyphenylamine containing oligomers and trace impurities, including the formamide of the methylene bridged polyphenylamine, with a hydrogenation catalyst, other than rhodium, and which is not a catalyst poison to rhodium, under conditions sufficient to effect partial hydrogenation of the reaction mixture and then, after this pretreatment or initial contact, catalytically hydrogenating the polyphenylamine to the polycyclohexylamine counterpart in the presence of a rhodium catalyst in a second hydrogenation zone. The initial hydrogenation of the crude methylene bridged polyphenylamine is typically carried out with ruthenium as the catalyst and the initial hydrogenation is sufficient to destroy the traces of formamide components in the reaction mixture and to prevent rapid deactivation of the rhodium catalyst during the secondary catalytic hydrogenation.

There are several advantages associated with this process. These include:
- an ability to extend the life of the rhodium catalyst for the catalytic hydrogenation of the phenyl groups to the cyclohexyl counter parts in crude methylene-bridged polyphenylamines;
- an ability to produce a hydrogenated methylenedianiline having a trans,trans- isomer concentration of 40% and less;
- an ability to effect hydrogenation of methylenedianiline and other methylene-bridged polyphenylamines at relatively low pressures e.g. 103,4 bar (1500 psig) and lower, and at excellent reaction rates;
- an ability to utilize an impure feeds, e.g. , one containing oligomers and trace amounts of formamide derivatives of methylene bridged polyphenylamines as a reactant and yet obtain cyclohexyl counterparts in high selectivity;
- an ability to obtain a reaction product which is substantially free of by-product oligomers and other heavies; and
- an ability to use the catalyst for continued periods of time with only modest maintenance or regeneration techniques.

The process of this invention involves the catalytic hydrogenation of methylene bridged polyphenylamines to the polycyclohexylamine counterparts utilizing a rhodium catalyst. These methylene bridged polyphenylamines are typically prepared by condensing an aromatic amine with formaldehyde to produce the methylene bridged polyphenylamine. Depending upon the condensation process, methylenediphenylamines and diphenyl diamines are produced with minor coproduction of oligomers; i.e., polyphenylamines containing three or more phenyl groups, typically 3 to 5 phenyl groups. Additionally, in view of the fact that formaldehyde is used to condense the amines, some additional by-products also produced in minor amount, with the major impurities being N-methylmethylenediphenylamine and the formamide of methylenediphenylamine. It is believed that because of these oligomers and other impurities in crude methylene-bridged polyphenylamines, rhodium is deactivated during the hydrogenation process. Representative methylene bridged phenylamines, which can be catalytically hydrogenated in accordance with the process of the invention, are set forth in the formula: 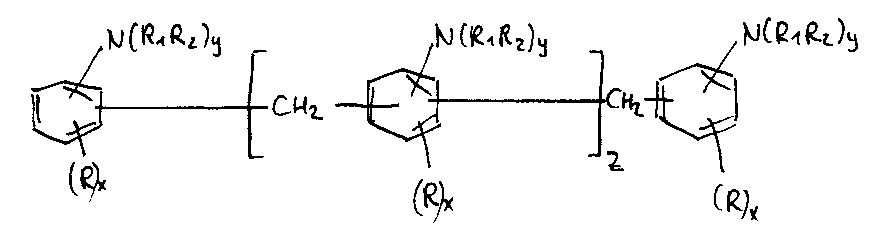
wherein R is H, C₁₋₆ alkyl, C-O-R₃; where R₃ is C₁₋₄ alkyl, CH₂NH₂, R₁ and R₂, H or C₁₋₆ alkyl, x is 1-4 y is 0 to 2 with the sum being at least 1, provided x + y is not greater than 5, and z is from 0 to 3; Representative compositions described by the formula include methylenedianiline; 3,3'-dimethyl-4,4'-diamino-diphenylmethane; 3,3'_{,}5,5'-tetramethyl-4,4'diaminodi-phenylmethane and 3,3'6,6' tetramethyl-4,4'-diaminodi-phenylmethane; 3,3'-ditertbutyl-4,4'-diaminodiphenylmethane and 3 3'-dimethyl-5,5'-ditertbutyl-4,4'-diaminodiphenylmethane; N,N'-dimeihyl-4,4'diaminodl-phenylmethane, tetramethyl-4,4'-diaminodi-phenylmethane.

The process involves the pretreatment or initial hydrogenation of a crude polyphenylamine feedstock containing oligomers and trace impurities, such as the formamide of the methylene-bridged polyphenylamines, with a hydrogenation catalyst other than rhodium and which is not poisonous to rhodium, under conditions sufficient for effecting partial hydrogenation of the feedstock. This initial hydrogenation typically is conducted with a ruthenium catalyst and is carried out for a time sufficient to effect at least substantial decomposition of the formamide impurity in the feedstock. The catalyst usually is incorporated in an amount from 0.0005 to 10% by weight as metal of the methylene-bridged polyphenylamine. The initial catalytic hydrogenation must be carried out for a time sufficient to remove sufficient impurities such that substantial deactivation of the rhodium catalyst does not occur during the secondary catalytic hydrogenation of the phenyl groups to the cyclohexyl counterparts. In this way the rhodium catalyst may be used repeatedly for the secondary catalytic hydrogenations without regeneration. Without such initial hydrogenation or pretreatment, the rhodium catalyst must be regenerated more frequently.

If analytical techniques are not available for monitoring the initial catalytic hydrogenation, a guide for initial hydrogenation would suggest that the catalytic hydrogenation using ruthenium catalyst would be in an amount of from about 0.0005 to 15% by weight (as metal) of the methylene bridged polyphenylamine, a reaction time of from 10 to 120 minutes, typically 30-60 minutes, at temperatures of 130 to 220°C. Shorter periods of time or lower hydrogenation temperatures might result in less removal of trace impurities and also result in slightly higher rates of rhodium catalyst deactivation in the hydrogenation process. If the initial catalytic hydrogenation is carried out over an extended period of time, some partial hydrogenation of the ring may occur and this simply extends the overall reaction time of the combined initial and secondary catalytic hydrogenation processes, but would have no detrimental effect upon catalyst life of rhodium. By effecting partial ring hydrogenation in the initial catalytic hydrogenation, one is assured that substantially all of the oligomers or impurities, or both, which are poisonous to the rhodium catalyst are destroyed and therefore catalyst life of rhodium is not adversely affected in the secondary hydrogenation.

As with conventional processes the hydrogenation process is carried out under liquid phase conditions, such liquid phase conditions being maintained typically by carrying out the hydrogenation in the presence of a solvent. Although as reported in the art, it is possible to hydrogenate the feedstock in the absence of a solvent, but the processing is much simpler when a solvent is employed. Representative solvents suited for practicing the invention include saturated aliphatic and alicyclic hydrocarbons such as cyclohexane, hexane, and cyclooctane; low molecular weight alcohols, such as methanol, ethanol, isopropanol; and aliphatic and alicyclic hydrocarbon ethers, such as n-propyl ether, isopropyl ether, n-butyl ether, amyl ether, tetrahydrofuran, dioxane, and dicyclohexylether. Tetrahydrofuran is preferred. Although in some processes water can be used as a cosolvent, it is preferred that the system be maintained in an anhydrous state or at least maintained so that the water concentration is less than 0.5% by weight.

When a solvent is used, it can be used in conventional amounts e.g. concentrations as low as 50% by weight, based on methylene-bridged polyphenylamine introduced into the reaction zone; typically the solvent is used at levels from about 75 to about 200% by weight of the starting polyphenylamine. Under some circumstances the solvent is used in amounts as high as 1000 to 2000% based upon the weight of the methylene bridged polyphenylamine.

The initial and secondary hydrogenations of the feed are carried out principally in a batch process although it is possible to operate the plant continuously. Temperatures used for the ring hydrogenation process range from about 130 to 220°C with preferred temperatures of from about 170 to 195°C. As with prior art hydrogenation processes, hydrogen partial pressures can range from about 34,5 to 275,8 bar (500 to 4000 psig). However, in contrast to prior art processes, hydrogen pressures can be as low as from about 48,3 to 103,4 bar (700 to 1500 psig), while retaining practical or commercially acceptable reaction rates, the latter processing pressures being preferred for lower equipment and operating costs. Such conditions for hydrogenation using a rhodium catalyst are described in U.S. 3,856,862 and U.S. 3,591,635 and are incorporated by reference.

The ability to hydrogenate methylene bridged polyphenylamines, particularly methylenedianiline, at low hydrogen partial pressures is expanded through the utilization of the pretreatment and a rhodium catalyst preferably a mixed rhodium and ruthenium catalyst system. The rhodium catalyst is broadly used in an amount to provide from 0.001 to 10% by weight of the aromatic amine. This mixed catalyst system permits kinetic control of the isomer mixtures produced by the reaction at low pressures, the ease of reaction being unexpectedly superior to the ease of reaction noted with the rhodium catalyst alone in an unpurified methylene bridged polyphenylamine feed. The catalysts can be added to the reactor individually or they may be physically admixed or combined and used as a single component. To simplify preparation and processing it is preferred to admix the two catalysts and incorporate them into the reaction medium as an admixture. The catalysts are combined, based upon their weights as metal, in a ratio of about 1 to 12 weight parts rhodium per weight part of ruthenium, preferably 4 to 8 parts rhodium per part ruthenium. As the concentration of rhodium increases vis-a-vis ruthenium the activity of the catalyst system increases and therefore lower temperatures or catalyst concentrations may be satisfactory.

The catalysts used and practiced in this invention generally are supported upon an inert carrier and representative carriers include carbon, calcium carbonate, rare earth oxides, such as cerium, praseodymium, or lanthanum; rare earth oxides or carbonates; alumina; barium sulfate; kieselguhr; pumice; titania; diatomaceous earth; and alkaline earth components such as calcium sulfate, calcium oxide, barium oxide, and barium sulfate. Preferred support materials are alumina and titania with titania being preferred. The catalyst usually comprises from about 1 to 25 weight parts metal/weight part of support.

To maintain high activity of the catalyst system in the hydrogenation process it is proposed that at least the rhodium component of the catalyst be alkali moderated. Alkali moderation techniques to produce the catalyst system are well known and the techniques disclosed in U.S. 3,636,108 for the alkali moderation of ruthenium can be utilized for the production of rhodium. Such method is incorporated by reference. Typically, such-alkali moderation involves the treatment of the catalyst and support material with an alkali metal hydroxide such as, sodium, lithium or potassium hydroxide or alkali metal alkoxide such as sodium, lithium, or potassium methoxide or ethoxide in an amount to provide from 0.1 to 15% by weight of a basic metal compound calculated as alkali metal. Often, moderation of the catalyst is done prior to reduction of the catalyst with aqueous dilute alkali metal hydroxide during or following metal deposition on the chosen support. Alkali moderation can also be accomplished in situ during hydrogenation by including alkali metal hydroxide, alkali metal alkoxide or by the addition of ammonia. For purposes of practicing this invention it is preferred that the catalyst is alkali moderated prior to reduction and maintained in situ with additions of alkali metal hydroxide.

The progress of the secondary hydrogenation reaction can readily be followed by observing the amount of hydrogen taken up by the reaction mixture. The reaction is terminated when the amount of hydrogen absorbed is equal to that amount necessary to effect complete hydrogenation of the product. In general, the hydrogenation time will range from about 45 to 900 minutes, at catalyst levels, e.g., 0.5-2.5% catalyst by weight of the methylene bridged polyphenylamine and generally will not exceed 300 minutes. The reaction time can be adjusted to adjust isomer selectivity as in the case of methylenedianiline.

The following examples are intended to illustrate various embodiments of the invention and all parts and percentages given are weight parts or weight percents unless otherwise specified.

### Example 1

### Crude Methylenedianiline (MDA) Pretreatment

A 300 cc autoclave was charged with a solution of 62.4 g of undistilled, i.e., crude methylenedianiline (MDA) [(85% MDA, 15% MDA oligomers, and trace amounts of the formamide of methylenedianiline and other by-products)] in 87.6 g of tetrahydrofuran (THF) and 0.1 g of catalyst consisting of 5% ruthenium on alumina. The autoclave was purged with nitrogen followed by hydrogen. The autoclave was then pressurized to about 48,3 bar (700 psig) with hydrogen and heated to 192°C. After this temperature was reached, the autoclave was maintained at 58,6 bar (850 psig) by addition of hydrogen from a ballast tank. The autoclave was held at 192°C for 100 min. Very little hydrogen had been consumed in this time. The autoclave was then cooled and vented. The GC analysis of the reactor effluent showed about 6% of the MDA had one ring hydrogenated. Neither the formamide of methylenedianiline nor fully hydrogenated methylenedianiline was seen in the product.

### Example 2

### Comparison Effect of Pretreatment

In this example 5 runs were made for comparison purposes. Run A illustrates the effectiveness of Rh and Run B illustrates the effectiveness of a Rh/Ru catalyst with the initially pretreated or hydrogenated feed of Example I. Runs C and D illustrate the limited effectiveness of Rh and Rh/Ru catalysts with crude MDA i.e., MDA which has not been pretreated and therefore containing trace oligomers and the formamide of methylenedianiline. Run E illustrates pretreatment of crude MDA feed with a Ni hydrogenation catalyst.

In Runs A and B a 300 cc autoclave was charged with 125.6 g of the effluent from Example 1. The catalyst was added along with 0.07 g of 10% by weight LiOH in water. The autoclave was sealed and purged with nitrogen followed by hydrogen addition. The autoclave was then pressurized to about 48,3 bar (700 psig) with hydrogen and heated to 192°C. At 192°C, the autoclave pressure was kept at 58,6 bar (850 psig) by addition of hydrogen from a ballast tank. In both Runs A and B hydrogen uptake was noticed by the time the temperature of 192°C was reached. The autoclave was cooled after the reaction was estimated to be completed.

For runs C & D, a 300 cc autoclave was charged with 125 g of 42% by weight crude MDA. The catalyst, along with 0.07 g of 10% by weight LiOH in water, were added. The autoclave was sealed and flushed with nitrogen followed by hydrogen. The autoclave was pressurized to about 41,4 bar (600 psi) with hydrogen was was heated to 192°C with agitation. Once the temperature of 192°C was reached, the autoclave pressure was maintained at 58,6 bar (850 psig) by addition of hydrogen throughout the reaction from a ballast tank. With the Rh catalyst, no hydrogen uptake was noticed for about 400 min. About 20% of the theoretical hydrogen was consumed in about 500 min. The reaction was complete in 920 min. With the Rh/Ru catalyst, the 10% theoretical hydrogen uptake took 36 min. and the reaction was complete in 200 min.

As for Run E, a 300 cc autoclave was charged with a crude MDA/THF solution as in Example 1. Then, 1 g of Raney Ni 2800 was added and the autoclave sealed and purged with nitrogen. This was followed by addition of hydrogen. The reactor was pressurized with hydrogen to about 48,3 bar (700 psig) then heated to 200°C. Hydrogen pressure was maintained at 58,6 bar (850 psig) by addition of hydrogen from a ballast tank. About 10% of the theoretical hydrogen was consumed in 2 hr at which time the reaction had ceased. GC analysis of the reactor effluent showed about 23% of the MDA had one ring hydrogenated. No MDA formamide derivative were noted in the initially hydrogenated feed. The reactor was filtered to remove the Ni catalyst. The filtrate was charged into a clean 300 cc autoclave along with 0.73 g of 5% Rh on alumina and 0.09 g of 5% Ru on alumina. Then 0.08 g of 10% LiOH in water was added and the autoclave sealed and purged with nitrogen. After purging hydrogen was added. The autoclave was pressurized with hydrogen to about 48,3 bar (700 psig) then heated to 192°C. Hydrogen uptake was noticed at about 160°C and the hydrogen ballast tank was opened to maintain the autoclave pressure at 58,6 bar (850 psig). The rate of hydrogen uptake dropped rapidly and became only one-fourth the initial rate in 45 minutes. The reaction was terminated after 300 minutes even though only 91% of the expected hydrogen uptake had occurred. The failure of the reaction to complete was verified by analysis of the product which showed 18% half hydrogenated MDA. Hydrogen uptake slowed rapidly and had apparently ceased after about 300 min. Results of Runs A-E are included in Table 1.

**Table 1**

| Feed | Catalyst | Pretreatment | Reaction Time(min) | % Conversion* | % Yield** | % t,t Isomer |
|---|---|---|---|---|---|---|
| Run A | 0.72 g Rh# | Yes | 250 | 100 | 87.5% | 23.2 |
| Run B | 0.72 g Rh# | Yes | 145 | 93 | 89.8 | 14.1 |
| | 0.09 g Ru# | | | | | |
| Run C | 0.72 g Rh# | No | 920 | 100 | 91.0 | 28.9 |
| Run D | 0.72 g Rh# | No | 200 | 100 | 91.2 | 16.1 |
| | 0.09 g Ru# | | | | | |
| Run E | 0.72 g Rh# | No | 300## | 91 | 77.8 | 28.5 |
| | 0.09 g Ru# | | | | | |

| | | | | | | |
|---|---|---|---|---|---|---|
| * Conversion based on observed consumption of hydrogen versus theory | | | | | | |
| ** Yield as PACM and half hydrogenated MDA based on MDA in feed | | | | | | |
| # Catalysts were 5% metal on an alumina support | | | | | | |
| ## Reaction had stopped | | | | | | |

As can be seen from the above data, excellent reaction rates were obtained through the use of a rhodium catalyst (Run A) and through the use of a mixed Rh/Ru metal catalyst (Run B) where the feed was initially pretreated or hydrogenated as compared to a non-pretreated feed. Compare Runs A and C and Runs B and D. Run B, which was terminated early (93% conversion), shows the mixed metal catalyst was more effective than the Rh catalyst alone. The nickel pretreatment in Run E was effective in reducing contaminants, e.g., the formaldehyde of MDA in the crude MDA feed but the Ni catalyst adversely affected the reactivity of the Rh/Ru catalyst system or poisoned it to the point where the catalyst was ineffective in the secondary hydrogenation step. As a result, the reaction failed to complete leaving 18% half reduced MDA.

### Example 3

### Catalyst Deactivation Study

This example illustrates the improved retention of catalyst activity during reuse when the crude MDA feed is pretreated or initially hydrogenated using a hydrogenation catalyst which is not a catalyst poison to rhodium, e.g. Ru.

A pretreated feed used in this example was prepared by the technique used in Example 1. A crude MDA/THF solution (2000 g) was charged into a 3,8 ℓ (1 gal) autoclave along with 12.5 g of 5% Ru on alumina catalyst. After nitrogen and hydrogen purging, the autoclave was heated to 195°C under 58,6 bar (850 psig) pressure by hydrogen addition. The temperature was maintained for 30 min after which the autoclave was cooled and vented. The reactor effluent was filtered to remove the catalyst. Secondary hydrogenations were made reusing a single charge of catalyst (1.5% catalyst based on the weight of crude MDA). In a first series of 7 runs the pretreated crude MDA/THF feed was hydrogenated with a catalyst which was 80% by weight of 5% Rh on alumina and 20% of 5% Ru on alumina. In a second series of 7 runs, crude MDA/THF which had not been pretreated or initially hydrogenated was hydrogenated with a catalyst which was 89% by weight of 5% Rh on alumina and 11% of 5% Ru on alumina. The reactions were run at approximately 180°C until completion as estimated by hydrogen uptake. The results are shown in Table 2.

**Table 2**

| Pretreated | Use | Reaction Time* | Conversion** | Yield# | t,t Isomer |
|---|---|---|---|---|---|
| Yes | 1 | 200 min | 99% | 88.2% | 19.2% |
| Yes | 2 | 240 | 99 | 87.8 | 19.2 |
| Yes | 3 | 190 | 100 | 89.2 | 25.1 |
| Yes | 4 | 190 | 99 | 92.8 | 21.4 |
| Yes | 5 | 230 | 100 | 93.8 | 21.8 |
| Yes | 6 | 230 | 98 | 96.1 | 21.2 |
| Yes | 7 | 245 | 98 | 94.9 | 21.9 |
| No | 1 | 300 | 100 | 88.3 | 21.5 |
| No | 2 | 330 | 90 | 91.6 | 14.3 |
| No | 3 | 370 | NO ANALYSIS | | - |
| No | 4 | 405 | 100 | 93.9 | 20.1 |
| No | 5 | 510 | 100 | 96.5 | 20.4 |
| No | 6 | 585 | 90 | 98.9 | 21.2 |
| No | 7 | 720 | 100 | 98.8 | 26.9 |

| | | | | | |
|---|---|---|---|---|---|
| * Time at 180°C, reaction may not be complete | | | | | |
| ** Conversion based on hydrogen consumption versus theory | | | | | |
| # Yield based on available MDA, includes PACM and one ring reduced MDA | | | | | |

As can be seen from the data in Table 2 pretreatment of crude MDA to remove the formamide of MDA and possibly other contaminants via an initial hydrogenation substantially enhanced rhodium catalyst life as compared to hydrogenation of crude MDA containing oligomers and formamide using a similar rhodium containing catalyst. Thus, the pretreatment improves reactivity of the catalyst and clearly extends catalyst life.

Although not intending to be bound by theory it is believed the initial pretreatment or hydrogenation is effective because it destroys an impurity in the feed. It is believed this impurity is the formamide of MDA and it acts as a poison to Rh. Besides inhibiting the Rh catalyst, this impurity most likely causes loss in Rh activity which is not reversed under reaction conditions. This deactivation is believed to be due to Rh oxidation.

## Claims

1. Process for the hydrogenation of a crude methylene bridged polyphenylamine to produce the corresponding methylene bridged polycyclohexylamine in the presence of a rhodium hydrogenation catalyst at a temperature and for a time sufficient to effect hydrogenation of the phenyl ring to the cyclohexyl ring, characterised by
(a) pretreating the crude methylene bridged polyphenylamine feed by an initial hydrogenation step in a first hydrogenation zone in the presence of hydrogen and a hydrogenation catalyst other than rhodium under conditions to partially hydrogenate the feed, said hydrogenation catalyst being non-poisonous to rhodium under said hydrogenation conditions, and then
(b) catalytically hydrogenating the methylene bridged polyphenylamine in the presence of a rhodium catalyst in a secondary hydrogenation zone for producing the corresponding methylene bridged polycyclohexylamine.

2. The process of Claim 1 wherein said methylene bridged-polyphenyl amines are represented by the formula 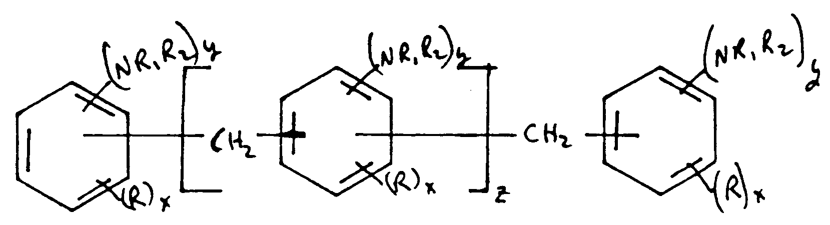 wherein R is H, C₁₋₆ alkyl, C-O-R₃; where R₃ is C₁₋₄ alkyl, CH₂NH₂, R₁ and R₂, H or C₁₋₆ alkyl, x is 1-4, y is 0 to 2 with the sum being at least 1, provided x + y is not greater than 5, and z is from 0 to 3.

3. The process of Claim 2 wherein said initial hydrogenation is carried out using ruthenium as the hydrogenation catalyst.

4. The process of Claim 3 wherein said initial hydrogenation is carried out at at temperature from 130 to 220°C and for a time from 10 to 120 minutes.

5. The process of Claim 4 wherein the ruthenium, as metal, is present in an amount of from 0.0005 to 10% by weight as metal of the methylene bridged polyphenylamine.

6. The process of Claim 5 wherein each x is 1 and each y is 1.

7. The process of Claim 6 wherein R₁ and R₂ are hydrogen.

8. The process of Claim 7 wherein R is hydrogen or methyl.

9. The process of Claim 8 wherein z is 0.

10. The process of Claim 9 wherein R is hydrogen.

11. The process of Claim 10 wherein the rhodium catalyst is present in an amount of from 0.001 to 10% by weight as metal of the methylene bridged polyphenylamine.

12. The process of claim 11 wherein the rhodium-containing catalyst system in the secondary hydrogenation zone comprises from 1 to 12 weight parts rhodium per weight part ruthenium.

13. The process of claim 12 wherein said rhodium is present in an amount to provide from 0,001 to 10 % metal by weight of the aromatic amine.

14. The process of claim 12 wherein the aromatic amine is 3,3'-di-tert-butyl-4,4'-diaminodiphenylmethane and the rhodium containing catalyst contains from 1 to 12 weight parts rhodium per weight part ruthenium.

15. The process of claim 12 wherein the aromatic amine is 3,3'-dimethyl-5,5'-di-tert-butyl-4,4'-diaminodiphenylmethane and the rhodium-containing catalyst contains from 1 to 12 weight parts rhodium per weight part ruthenium.

## Patentansprüche

1. Verfahren zur Hydrierung eines rohen, methylenverbrückten Polyphenylamins zur Herstellung des entsprechenden methylenverbrückten Polycyclohexylamins in Gegenwart eines Rhodium-Hydrierkatalysators bei einer Temperatur und für eine Zeit, die zur Hydrierung des Phenylrings zum Cyclohexylring ausreicht, dadurch gekennzeichnet, daß man
(a) die rohe Beschickung des methylenverbrückten Polyphenylamins in einem anfänglichen Hydrierschritt in einer ersten Hydrierzone in Gegenwart von Wasserstoff und in Gegenwart eines Hydrierkatalysators, der ein anderer Katalysator als Rhodium ist, unter Bedingungen vorbehandelt, um die Beschickung partiell zu hydrieren, wobei der Hydrierkatalysator unter den Hydrierbedingungen für Rhodium nicht giftig ist, und danach
(b) das methylenverbrückte Polyphenylamin in Gegenwart eines Rhodiumkatalysators in einer zweiten Hydrierzone zur Herstellung des entsprechenden methylenverbrückten Polycyclohexylamins katalytisch hydriert.

2. Verfahren nach Anspruch 1, worin die methylenverbrückten Polyphenylamine durch die folgende Formel wiedergegeben werden 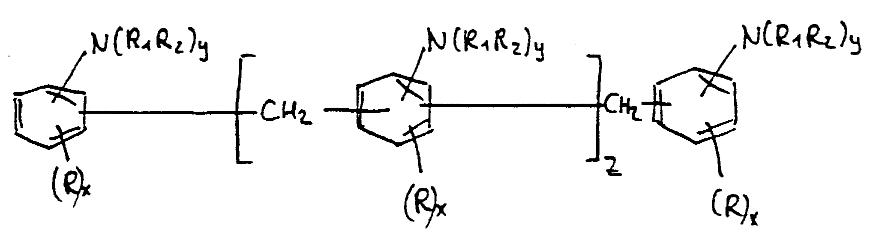 worin R für H, C₁₋₆-Alkyl, C-O-R₃ steht, worin R₃ für C₁₋₄-Alkyl und CH₂NH₂ steht, R₁ und R₂ für H oder C₁₋₆-Alkyl stehen, x für 1 bis 4 steht, y für 0 bis 2 steht, wobei die Summe wenigstens 1 ist, mit der Maßgabe, daß (x + y) nicht größer als 5 ist, und z für 0 bis 3 steht.

3. Verfahren nach Anspruch 2, worin die anfängliche Hydrierung unter Verwendung von Ruthenium als Hydrierkatalysator durchgeführt wird.

4. Verfahren nach Anspruch 3, worin die anfängliche Hydrierung bei einer Temperatur von 130 bis 220 °C und für eine Zeit von 10 bis 120 Minuten durchgeführt wird.

5. Verfahren nach Anspruch 4, worin das Ruthenium, angegeben als Metall, in einer Menge von 0,0005 bis 10 Gew.-%, angegeben als Metall und bezogen auf das methylenverbrückte Polyphenylamin, zugegen ist.

6. Verfahren nach Anspruch 5, worin jedes x für 1 steht und jedes y für 1 steht.

7. Verfahren nach Anspruch 6, worin R₁ und R₂ Wasserstoff sind.

8. Verfahren nach Anspruch 7, worin R Wasserstoff oder Methyl ist.

9. Verfahren nach Anspruch 8, worin z 0 ist.

10. Verfahren nach Anspruch 9, worin R Wasserstoff ist.

11. Verfahren nach Anspruch 10, worin der Rhodiumkatalysator in einer Menge von 0,001 bis 10 Gew.-%, angegeben als Metall und bezogen auf das methylenverbrückte Polyphenylamin, zugegen ist.

12. Verfahren nach Anspruch 11, worin das Rhodium enthaltende Katalysatorsystem in der Sekundärhydrierzone 1 bis 12 Gew.-Teile Rhodium pro Gew.-Teil Ruthenium umfaßt.

13. Verfahren nach Anspruch 12, worin das Rhodium in einer Menge zugegen ist, um 0,001 bis 10 Gew.-% Metall, bezogen auf das aromatische Amin, bereitzustellen.

14. Verfahren nach Anspruch 12, worin das aromatische Amin 3,3'-Di-tert-butyl-4,4'-diaminodiphenylmethan ist und der Rhodium enthaltende Katalysator 1 bis 12 Gew.-Teile Rhodium pro Gew.-Teil Ruthenium enthält.

15. Verfahren nach Anspruch 12, worin das aromatische Amin 3,3'-Dimethyl-5,5'-di-tert-butyl-4,4'-diaminodiphenylmethan ist und der Rhodium enthaltende Katalysator 1 bis 12 Gew.-Teile Rhodium pro Gew.-Teil Ruthenium enthält.

## Revendications

1. Procédé pour l'hydrogénation d'une polyphénylamine brute à pontage méthylène, pour produire la polycyclohexylamine à pontage méthylène correspondante, en présence d'un catalyseur d'hydrogénation au rhodium, à une température et pendant un temps suffisants pour effectuer l'hydrogénation du cycle phényle en le cycle cyclohexyle, caractérisé par
(a) le prétraitement de l'alimentation de polyphénylamine brute à pontage méthylène par une étape d'hydrogénation initiale dans une première zone d'hydrogénation, en présence d'hydrogène et d'un catalyseur d'hydrogénation autre que le rhodium, dans des conditions hydrogénant partiellement l'alimentation, ledit catalyseur d'hydrogénation n'empoisonnant pas le rhodium dans lesdites conditions d'hydrogénation, puis
(b) l'hydrogénation catalytique de la polyphénylamine à pontage méthylène, en présence d'un catalyseur au rhodium, dans une zone d'hydrogénation secondaire, pour produire la polycyclohexylamine à pontage méthylène correspondante.

2. Le procédé de la revendication 1, où lesdites polyphénylamines à pontage méthylène sont représentées par la formule 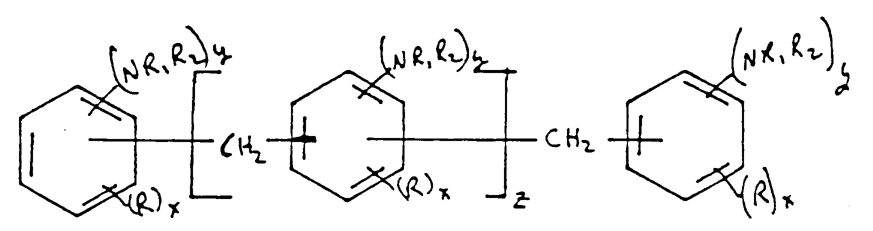 dans laquelle R est H, un alkyle en C₁₋₆, C-O-R₃ où R₃ est un alkyle en C₁₋₄ ou CH₂N₂, R₁ et R₂ sont H ou un alkyle en C₁₋₆, x est 1-4, y est 0 à 2, la somme étant d'au moins 1, sous réserve que x + y ne soit pas supérieur à 5 et z est de 0 à 3.

3. Le procédé de la revendication 2, où ladite hydrogénation initiale est effectuée en utilisant du ruthénium comme catalyseur d'hydrogénation.

4. Le procédé de la revendication 3 où ladite hydrogénation initiale est effectuée à une température de 130 à 220°C pendant un temps de 10 à 120 minutes.

5. Le procédé de la revendication 4 où le ruthénium, en métal, est présent en une proportion de 0,0005 à 10 % en poids en métal relativement à la polyphénylamine à pontage méthylène.

6. Le procédé de la revendication 5 où chaque x est 1 et chaque y est 1.

7. Le procédé de la revendication 6 où R₁ et R₂ sont un hydrogène.

8. Le procédé de la revendication 7 où R est un hydrogène ou un méthyle.

9. Le procédé de la revendication 8 où z est 0.

10. Le procédé de la revendication 9 où R est un hydrogène.

11. Le procédé de la revendication 10 où le catalyseur au rhodium est présent en une proportion de 0,001 à 10 % en poids en métal relativement à la polyphénylamine à pontage méthylène.

12. Le procédé de la revendication 11 où le système catalytique contenant du rhodium, dans la seconde zone d'hydrogénation secondaire, contient de 1 à 12 parties en poids de rhodium par partie en poids de ruthénium.

13. Le procédé de la revendication 12 où ledit rhodium est présent en une quantité apportant 0,001 à 10 % de métal relativement au poids de l'amine aromatique.

14. Le procédé de la revendication 12 où l'amine aromatique est le 3,3'-di-tert-butyl-4,4'-diaminodiphénylméthane et le catalyseur contenant du rhodium contient de 1 à 12 parties en poids de rhodium par partie en poids de ruthénium.

15. Le procédé de la revendication 12 où l'amine aromatique est le 3,3'-diméthyl-5,5'-di-tert-butyl-4,4'-diaminodiphénylméthane et le catalyseur contenant du rhodium contient de 1 à 12 parties en poids de rhodium par partie en poids de ruthénium.
